# EUROPEAN PATENT SPECIFICATION

(11) **EP 4 482 536 B1**
(45) Date of publication and mention of the grant of the patent: **02.09.2026**
(21) Application number: 23716355.5
(22) Date of filing: 23.02.2023
(51) Int. Cl.: A61L 2/12, A61L 9/18, H05B 6/00

(54) **MICROWAVE DISINFECTION PROTOCOL OPTIMISED FOR THE DESTRUCTION/ INACTIVATION OF SARS-COV-2 AND H1N1 VIRUSES**
MIKROWELLENDESINFEKTIONSPROTOKOLL OPTIMIERT FÜR DIE ZERSTÖRUNG/INAKTIVIERUNG VON SARS-COV-2 UND HINT-VIREN
PROTOCOLE DE DÉSINFECTION PAR MICRO-ONDES OPTIMISÉ POUR LA DESTRUCTION/L'INACTIVATION DES VIRUS SARS-COV-2 ET HINT

(30) Priority: 23.02.2022 EP 22158343
(43) Date of publication of application: 01.01.2025
(73) Proprietor: Elettronica S.p.A., 00131 Roma (IT)
(72) Inventor: MANNA, Antonio, 00131 ROMA (IT); PASCULLI, Nicola, 00131 ROMA (IT); BIA, Pietro, 00131 ROMA (IT); BARTOCCI, Marco, 00131 ROMA (IT); CUCCHIELLA, Cristian, 00131 ROMA (IT)
(74) Representative: Studio Torta S.p.A.
(86) International application number: PCT/IB2023/051661
(87) International publication number: WO 2023/161837

(56) References cited:
- US-A1- 2021 299 289

## Description

### Technical Field of the Invention

The present invention concerns, in general, a microwave disinfection device based on an innovative electromagnetic field application protocol.

More specifically, the present invention relates to a device configured to destroy and/or inactivate the SARS-CoV-2 virus and the H1N1 influenza virus by microwave irradiation using an optimised operation protocol characterised by low field intensity, appropriate operating frequency bands and appropriate microwave emission timing parameters, as well as by appropriate shapings of the irradiated beams.

### Background Art

International application WO 2022/018772 A1 of the Applicant relates to a microwave disinfection system and method.

In particular, the microwave disinfection system according to WO 2022/018772 A1 is configured to destroy and/or inactivate in real time pathogens (i.e., microorganisms that cause or have the ability to cause, or are responsible for the onset of, a disease in the human body, such as for example viruses, coronaviruses, viroids, germs, bacteria, mycetes (i.e., fungi), moulds, protozoa, prions, etc.) by microwave irradiation and related microwave resonant absorption by the pathogens.

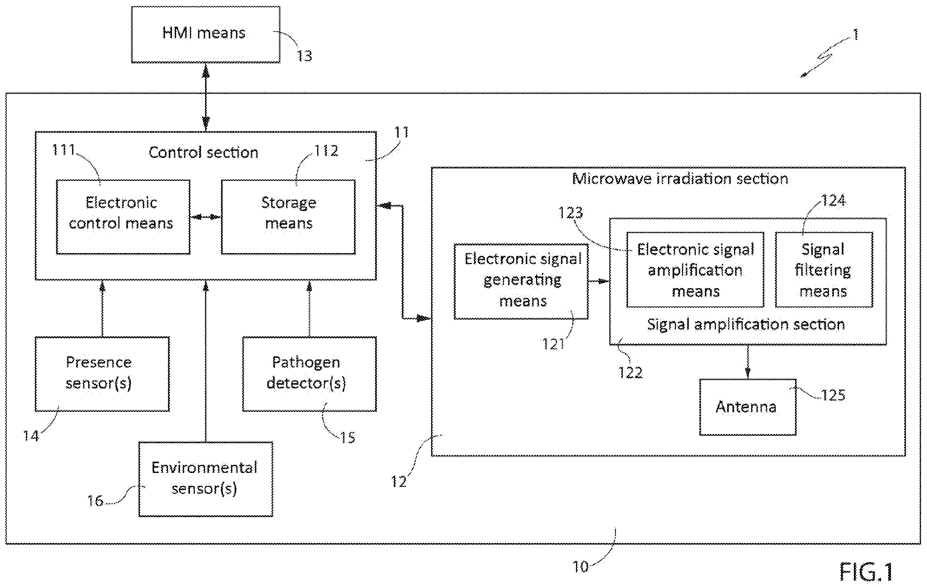

In this regard, Figure 1 shows schematically a high-level architecture of the microwave disinfection system according to WO 2022/018772 A1 (denoted as a whole by 1).

As shown in Figure 1, the microwave disinfection system 1 comprises a microwave disinfection device 10 that includes:
- a control section 11 that includes electronic control means 111 and storage means 112; and
- a microwave irradiation section 12 that includes
   - electronic signal generating means 121 controlled by the electronic control means 111,
   - a signal amplification section 122 which, in use, is driven by the electronic signal generating means 121 and is controlled by the electronic control means 111 and that includes electronic signal amplification means 123 and signal filtering means 124, and
   - an antenna 125 which, in use, is driven by the signal amplification section 122.

Furthermore, the microwave disinfection system 1 also comprises human-machine interface (HMI) means 13 which, in the example shown in Figure 1, are external to the microwave disinfection device 10 and are remotely connected, for example in wireless mode, to the control section 11.

More generally, the HMI means 13 could be connected to the control section 11 in wired or wireless mode, for example via connection based on USB technology (acronym for "Universal Serial Bus"), or via one or more Internet Protocol (IP)-based networks, e.g., the Internet network, and/or via one or more cellular telephone networks (e.g., GSM, GPRS, UMTS, HSPA, LTE, 4.5g, 5G, etc.) and/or one or more local, home, corporate, public, private networks, or via Bluetooth technology, etc.

Said HMI means 13 may be realized by means of a PC, a laptop, a tablet, a smartphone, a smartwatch, etc.

According to an alternative embodiment with respect to that shown in Figure 1, the HMI means 13 could instead be integrated into the microwave disinfection device 10, for example they could be realized in the form of one or more displays (such as of the touchscreen type) and/or one or more user interface devices (UI) of the hard and/or soft key type.

The control section 11 may be configured by a user by means of the HMI means 13 by:
- setting one or more predefined microwave irradiation parameters related to one or more predefined microwave disinfection treatments against one or more pathogens; and
- storing said predefined microwave irradiation parameters in the storage means 112.

In other words, the HMI means 13 are configured to allow a user to set up and store in the storage means 112 one or more predefined microwave irradiation parameters related to one or more predefined microwave disinfection treatments against one or more pathogens, thereby configuring the control section 11 to perform said predefined microwave disinfection treatment(s) against said pathogen(s) by means of the microwave irradiation section 12.

In particular, the HMI means 13 are configured to allow a user to set and store in the storage means 112, for a specific pathogen (conveniently, for each pathogen of a plurality of given pathogens), one or more respective predefined microwave irradiation parameter(s) related to one or more respective predefined microwave disinfection treatments against said specific pathogen, wherein said respective predefined microwave irradiation parameter(s) may include one or more of the following parameters related to microwave inactivation of said specific pathogen:
- one or more predefined microwave irradiation waveforms;
- one or more predefined wavelengths (i.e., one or more predefined frequencies) of microwave irradiation and/or one or more predefined wavelength bands (i.e., one or more predefined frequency bands) of microwave irradiation;
- one or more predefined microwave irradiation timing parameters (e.g., in terms of duration of each individual microwave irradiation and frequency of repetition of the microwave irradiations);
- one or more predefined microwave irradiation powers.

More specifically, the HMI means 13 are configured to store in the storage means 112, for the specific pathogen (conveniently, for each pathogen of said given pathogens), a respective library containing said respective predefined microwave irradiation parameter(s).

The electronic control means 111 are, therefore, configured to operate, i.e., to control the operation of, said microwave irradiation section 12 (in particular to operate, i.e., to control the operation of said electronic signal generating means 121 and said signal amplification section 122) on the basis of said predefined microwave irradiation parameter(s) stored in the storage means 112 so that said microwave irradiation section 12 performs said predefined microwave disinfection treatment(s) against said pathogen (s) .

More specifically, the microwave irradiation section 12 is configured to perform, when operated by the electronic control means 111, one or more microwave irradiations based on said predefined microwave irradiation parameter(s), i.e., to emit microwave signals in accordance with said predefined microwave irradiation parameter(s) (e.g., with the predefined waveform(s), and/or the predefined wavelength(s) or frequency(ies), and/or the predefined wavelength or frequency band(s), and/or the predefined timing parameter(s), and/or the predefined power(s)).

As shown in Figure 1, the microwave disinfection device 10 may also comprise one or more presence (or proximity) sensors 14 configured to:
- detect the presence of one or more persons near the microwave disinfection device 10 (e.g., within a predefined distance from the latter), i.e., in a given environment in which said microwave disinfection device 10 is installed/arranged; and
- signal the presence of one or more persons to the electronic control means 111, which can be configured to operate the microwave irradiation section 12 when the presence sensor(s) 14 detect(s) the presence of one or more persons.

In addition, the microwave disinfection device 10 may also comprises one or more pathogen detectors 15 configured to:
- detect the presence of one or more pathogens; and
- signal the presence of one or more pathogens to the electronic control means 111, which can be configured to operate the microwave irradiation section 12 based on the predefined microwave irradiation parameter(s) stored in the storage means 112.

The microwave disinfection device 10 may also comprise one or more environmental sensors 16 (e.g., a temperature sensor and/or a pressure sensor and/or a humidity sensor and/or one or more pollutant/harmful element/substance detectors, etc.) configured to send respective environmental monitoring data to the electronic control means 111, which may be configured to control the operation of the microwave irradiation section 12 based also on the environmental monitoring data received from the environmental sensor(s) 16.

### Object and Summary of the Invention

Starting from the microwave disinfection system according to WO 2022/018772 A1, the Applicant continued its scientific research carrying out a series of experimental tests in order to identify an optimised microwave disinfection protocol for the destruction/inactivation of the SARS-CoV-2 virus and of the H1N1 influenza virus, thus reaching the present invention.

Therefore, object of the present invention is to provide a microwave disinfection device specifically configured to destroy/inactivate the SARS-CoV-2 virus and the H1N1 influenza virus.

This and other objects are achieved by the present invention as it relates to a microwave disinfection device, as defined in the attached Claims.

In particular, the present invention concerns a microwave disinfection device configured to destroy/inactivate SARS-CoV-2 and H1N1 viruses and comprising a microwave irradiation section and a control section configured to control the operation of the microwave irradiation section so as to:
- irradiate microwave signals that have an incident electric field amplitude not higher than 6 V/m and frequencies that are included in the 8-10 GHz band and are spaced from each other by a range/step comprised between 10 MHz and 100 MHz;
- irradiate the microwave signals at each individual frequency for a time interval comprised between 50 ms and 1 s; and
- irradiate the microwave signals with duty cycle comprised between 5% to 50%.

### Brief Description of the Drawings

For a better understanding of the present invention, some preferred embodiments (provided purely by way of explanatory example, though not at all limiting, nor binding) will now be shown with reference to the accompanying drawings (not to scale), wherein:
- Figure 1 schematically shows a microwave disinfection system described in WO 2022/018772 A1;
- Figure 2 schematically shows a microwave disinfection device according to an embodiment of the present invention; and
- Figures 3 and 4 show two examples of embodiments of the microwave disinfection device in Figure 2.

### Description of Embodiments of the Invention

The following description is provided to enable a person skilled in the art to make and use the invention. Various modifications to the embodiments set forth will be immediately clear to the persons skilled in the art and the general principles herein disclosed may be applied to other embodiments and applications without, however, departing from the protection scope of the present invention as defined in the enclosed Claims. Therefore, the present invention should not be understood as limited to the sole embodiments described and shown, but it must be given the widest scope of protection in accordance with the characteristics defined in the appended Claims.

The present invention concerns a device configured to destroy and/or inactivate the SARS-CoV-2 virus and the H1N1 influenza virus by microwave irradiation using an optimised operation protocol characterised by low field intensity, appropriate operating frequency bands and appropriate microwave emission timing parameters, as well as by appropriate shaping of the irradiated beams. These features enable the realization of a device that is wearable or portable by a user that is capable of inactivating in real time viral particles of the SARS-CoV-2 and/or H1N1 type that are present in a volume of space around/near the device itself, i.e., in a volume of space around/near the user, ensuring compatibility with the user's presence. In fact, the irradiated radio frequency (RF) signal - more specifically, the microwave irradiated signal - is compatible with human presence and does not create interferences to other electronic devices present in the surrounding environment.

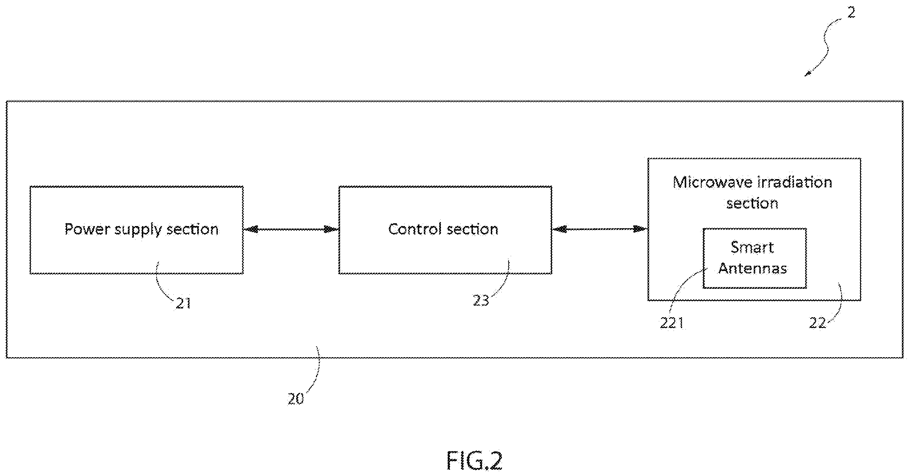

Figure 2 schematically shows an example of top level architecture of a microwave disinfection device (denoted as a whole by 2) according to a preferred (though not at all limiting, nor binding) embodiment of the present invention.

The microwave disinfection device 2 is realized in the form of a wearable device 20 (intended, therefore, to be worn by a user, not shown in Figure 2) and comprises:
- a power supply section 21 that includes a rechargeable battery (not shown in Figure 2);
- a microwave irradiation section 22 that is configured to generate and irradiate microwave signals and that includes smart antennas 221 for irradiating microwave signals; and
- a control section 23 configured to control, in general, the operation of the microwave disinfection device 2 (e.g. the switching on and off of the latter) and, in particular, the operation of the microwave irradiation section 22.

The control section 23 and the microwave irradiation section 22 of the microwave disinfection device 2 may conveniently have the same architecture, respectively, as the control section 11 and the microwave irradiation section 12 of the microwave disinfection system 1 according to WO 2022/018772 A1.

In addition, the control section 23 may be conveniently connected in wired or wireless mode and controlled by HMI means (not shown in Figure 2 - e.g. realized by means of a PC, a laptop, a tablet, a smartphone, a smartwatch, etc.) in accordance with what is described in WO 2022/018772 A1 regarding the HMI means 13 and the control section 11. The HMI means could also be integrated into the microwave disinfection device 2 itself (e.g. it could be realized in the form of one or more touchscreen type displays and/or one or more user interface devices of the hard and/or soft key type) .

The control section 23 is configured to operate, i.e., to control the operation of, said microwave irradiation section 22 on the basis of predefined microwave irradiation parameters so that said microwave irradiation section 22 performs one or more specific microwave disinfection treatments against the SARS-CoV-2 virus and the H1N1 influenza virus.

The microwave irradiation section 22 is, therefore, configured to, when operated by the control section 23, perform microwave irradiations based on said predefined microwave irradiation parameters, i.e., irradiate, by means of the smart antennas 221, microwave signals in accordance with said predefined microwave irradiation parameters.

More specifically, the predefined microwave irradiation parameters, which are specifically optimised for the destruction/inactivation of the SARS-CoV-2 virus and of the H1N1 influenza virus, include:
- frequencies of the irradiated microwave signals included in the 8-10 GHz band and spaced apart from each other by a range/step comprised between 10 MHz and 100 MHz;
- irradiation of microwave signals at each individual frequency for a time interval comprised between 50 ms and 1 s;
- irradiated microwave signals with an incident electric field amplitude not higher than 6 V/m;
- irradiation of the microwave signals performed with duty cycles comprised between 5% and 50%.

Therefore, in use, when operated by the control section 23, the microwave irradiation section 22 irradiates, by means of the smart antennas 221, microwave signals with frequencies ranging in the 8-10 GHz band and spaced from each other by a range/step ranging from 10 MHz to 100 MHz, irradiating the microwave signals at each individual frequency for a time interval ranging from 50 ms to 1 s, wherein the irradiated microwave signals have an incident electric field amplitude not higher than 6 V/m and wherein the irradiations of the microwave signals are performed with a duty cycle ranging from 5% to 50%.

The Applicant succeeded in identifying the aforesaid microwave irradiation parameters specifically optimised for the destruction/inactivation of the SARS-CoV-2 virus and of the H1N1 influenza virus by performing countless treatment tests in aerosols containing SARS-CoV-2 and H1N1. In particular, the tests made using the aforesaid microwave irradiation parameters produced inactivation results greater than 1 log (90%) for the SARS-CoV-2 virus and greater than 2 logs for the H1N1 influenza virus.

Moreover, thanks to the aforesaid microwave irradiation parameters, it is possible to realise a miniaturised and therefore wearable/transportable device for inactivation of viral particles in aerosol.

The smart antennas 221 are further configured to irradiate a predefined microwave beam shaped so that the microwave signals:
- are irradiated in a predefined volume of space (in which inactivation of the viral particles in aerosol takes place) near or around the microwave disinfection device 2; and
- are not irradiated directly on the user, i.e., towards the body of the user wearing said microwave disinfection device 2.

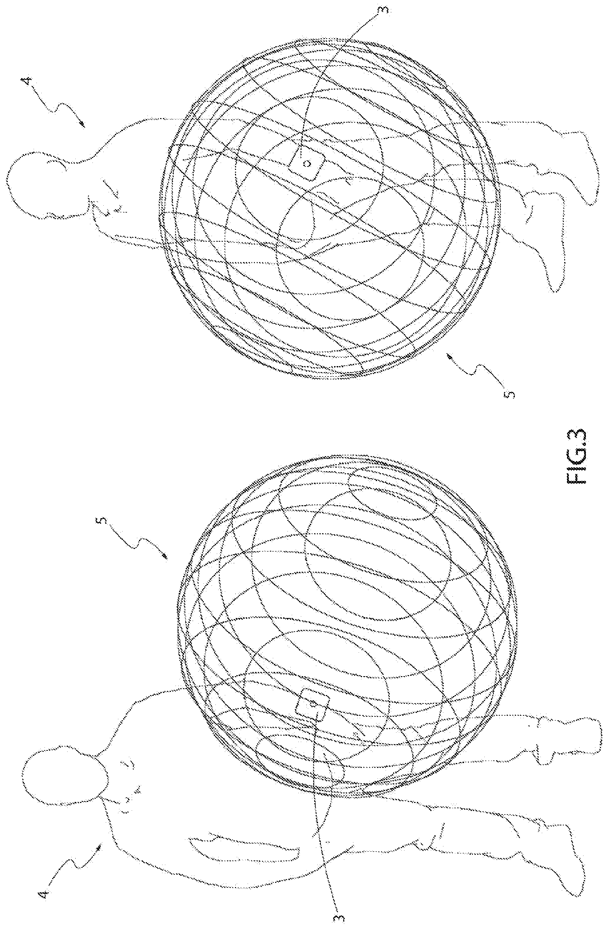

Figure 3 shows a first example of embodiment of the microwave disinfection device 2 in the form of a bracelet or smartwatch 3 worn by a user 4 (in particular on the latter's left wrist) and configured to emit a suitably shaped radiation beam 5 - in particular, shaped so as to create a region of inactivation of the SARS-CoV-2 and H1N1 viruses of substantially spherical shape on the left side of the user 4, externally to the latter's body.

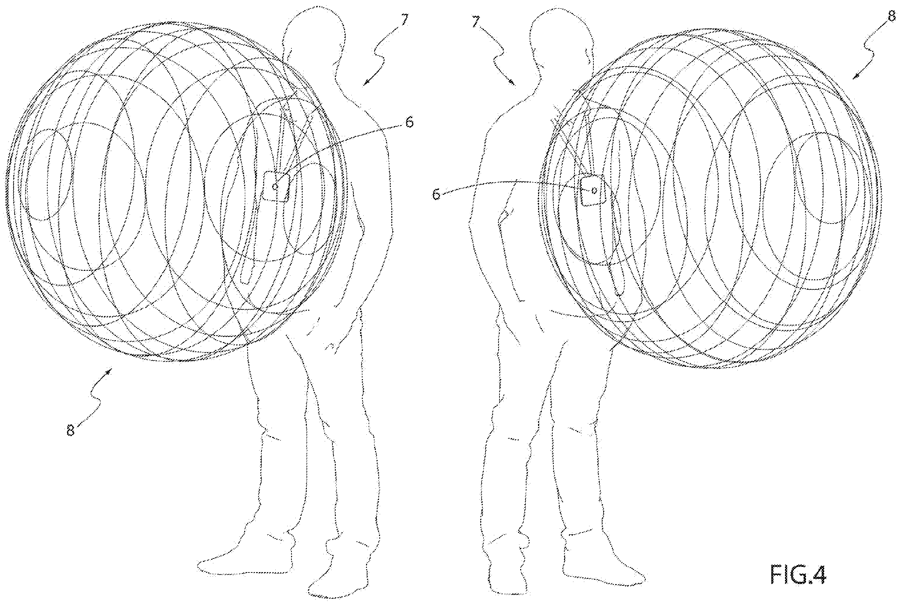

Furthermore, Figure 4 shows a second example of embodiment of the microwave disinfection device 2 in the form of a pendant 6 worn by a user 7 (in particular around the latter's neck) and configured to emit a suitably shaped radiation beam 8 - in particular, shaped so as to create a region of inactivation of the SARS-CoV-2 and H1N1 viruses of substantially spherical shape in front of the torso of the user 7, externally with respect to the latter's body.

The microwave disinfection device 2 could also be conveniently realized in the form of an accessory to be clipped onto the belt or carried in one's pocket.

More generally, the microwave disinfection device 2 could be conveniently realized in the form of, or integrated into, any portable or wearable object or device.

In addition, the microwave disinfection device according to the present invention can also be conveniently integrated into any electronic appliance both of the household type (e.g., refrigerators, washing machines, ovens, televisions, chandeliers or the like), and of the computer type (e.g., PC, tablet, smartphone, smartwatch or the like), or installed inside transport vehicles for both private and public use.

From the foregoing description, the innovative features and the technical advantages of the present invention are immediately clear to those skilled in the art.

In particular, it is important to emphasise that the present invention makes it possible to realize a device, preferably of the portable or wearable type, that is capable of inactivating in real time the SARS-CoV2 and H1N1 viruses by applying an innovative low-level electromagnetic field protocol that guarantees compatibility with human presence.

In conclusion, it is important to note that, while the above described invention refers in particular to very specific embodiments, it must not be intended as limited to such embodiments, including within its scope all the variants, modifications or simplifications covered by the enclosed Claims.

## Claims

1. Microwave disinfection device (2) configured to destroy/inactivate SARS-CoV-2 and H1N1 viruses and including a microwave irradiation section (22) and a control section (23) configured to control the operation of the microwave irradiation section (22) so as to:
• irradiate microwave signals that have an incident electric field amplitude not higher than 6 V/m and frequencies that are included in the 8-10 GHz band and are spaced from each other by a step comprised between 10 MHz to 100 MHz;
• irradiate the microwave signals at each individual frequency for a time interval comprised between 50 ms and 1 s; and
• irradiate the microwave signals with duty cycle comprised between 5% and 50%.

2. The microwave disinfection device of Claim 1, wherein the microwave irradiation section (22) includes smart antennas (221) and is configured to irradiate the microwave signals by means of said smart antennas (221), which are configured to irradiate a predefined microwave beam shaped so that the microwave signals are irradiated in a predefined volume of space near or around the microwave disinfection device (2).

3. The microwave disinfection device of Claim 2, realized in the form of, or configured to be integrated into, a device that is portable or wearable (20,3,6) by a user (4,7); wherein the predefined irradiated microwave beam in use by the smart antennas (221) is shaped so that the microwave signals are not radiated towards the user's body (4,7).

4. The microwave disinfection device according to any one of Claims 1-3, also comprising a power supply section (21) that includes a rechargeable battery.

5. The microwave disinfection device according to any one of Claims 1-4, configured to be integrated in an electronic device or transport vehicle.

## Patentansprüche

1. Mikrowellendesinfektionsvorrichtung (2), die ausgelegt ist, um SARS-CoV-2- und H1N1-Viren zu zerstören/zu inaktivieren, und die einen Mikrowellenabstrahlungsabschnitt (22) und einen Steuerabschnitt (23), der ausgelegt ist, um den Betrieb des Mikrowellenabstrahlungsabschnitt (22) zu steuern, umfasst, sodass:
• Mikrowellensignale abgestrahlt werden, die eine Amplitude des einfallenden elektrischen Felds aufweisen, die nicht höher als 6 V/m ist, und Frequenzen, die innerhalb des 8-10-GHz-Bands liegen und voneinander um eine Schrittweite im Bereich zwischen 10 MHz bis 100 MHz beabstandet sind;
• die Mikrowellensignale bei jeder einzelnen Frequenz für einen Zeitraum im Bereich zwischen 50 ms und 1 s abgestrahlt werden und
• die Mikrowellensignale mit einem Tastverhältnis im Bereich zwischen 5 % und 50 % abgestrahlt werden.

2. Mikrowellendesinfektionsvorrichtung nach Anspruch 1, wobei der Mikrowellenabstrahlungsabschnitt (22) Smart-Antennen (221) beinhaltet und ausgelegt ist, um Mikrowellensignale mittels der Smart-Antennen (221) abzustrahlen, die ausgelegt sind, um einen vordefinierten Mikrowellenstrahl abzustrahlen, der so ausgeformt ist, dass die Mikrowellensignale in einem vordefinierten Raumvolumen in der Nähe der Mikrowellendesinfektionsvorrichtung (2) oder um diese herum abgestrahlt werden.

3. Mikrowellendesinfektionsvorrichtung nach Anspruch 2, die in Form einer Vorrichtung, die durch einen Nutzer (4, 7) tragbar oder am Körper tragbar (20, 3, 6) ist, ausgebildet oder in diese integriert ist, wobei der vordefinierte Mikrowellenstrahl bei Gebrauch durch die Smart-Antennen (221) so ausgeformt ist, dass die Mikrowellensignale nicht in Richtung des Körpers des Nutzers (4, 7) abgestrahlt werden.

4. Mikrowellendesinfektionsvorrichtung nach einem der Ansprüche 1-3, zudem umfassend einen Stromversorgungsabschnitt (21), der einen wiederaufladbaren Akku beinhaltet.

5. Mikrowellendesinfektionsvorrichtung nach einem der Ansprüche 1-4, die ausgelegt ist, um in eine elektronische Vorrichtung oder ein Transportfahrzeug integriert zu werden.

## Revendications

1. Dispositif de désinfection par micro-ondes (2) configuré pour détruire/inactiver les virus SARS-CoV-2 et H1N1, et comprenant une section d'irradiation par micro-ondes (22) et une section de commande (23) configurée pour commander le fonctionnement de la section d'irradiation par micro-ondes (22) de manière à :
• irradier des signaux micro-ondes dont l'amplitude du champ électrique incident ne dépasse pas 6 V/m et dont les fréquences se situent dans la bande 8-10 GHz et sont espacées les unes des autres d'un pas compris entre 10 et 100 MHz ;
• irradier les signaux micro-ondes à chaque fréquence distincte pendant un intervalle de temps compris entre 50 ms et 1 s ; et
• irradier les signaux micro-ondes dont le cycle de service est compris entre 5 et 50 %.

2. Dispositif de désinfection par micro-ondes selon la revendication 1, la section d'irradiation par micro-ondes (22) comprenant des antennes intelligentes (221) et étant configurée pour irradier les signaux micro-ondes au moyen desdites antennes intelligentes (221), étant configurées pour irradier un faisceau de micro-ondes prédéfini formé de manière à ce que les signaux micro-ondes soient irradiés dans un volume d'espace prédéfini à proximité ou autour du dispositif de désinfection par micro-ondes (2).

3. Dispositif de désinfection par micro-ondes selon la revendication 2, réalisé sous la forme d'un appareil portable ou portable sur soi (20, 3, 6) par un utilisateur (4, 7), ou configuré pour être intégré à un tel appareil ; le faisceau de micro-ondes prédéfini irradié par les antennes intelligentes (221) étant formé de telle sorte que les signaux micro-ondes ne soient pas rayonnés vers le corps de l'utilisateur (4, 7).

4. Dispositif de désinfection par micro-ondes selon l'une quelconque des revendications 1-3, comprenant en outre une section d'alimentation (21) qui comporte une batterie rechargeable.

5. Dispositif de désinfection par micro-ondes selon l'une quelconque des revendications 1-4, configuré pour être intégré dans un appareil électronique ou un véhicule de transport.
